# EUROPÄISCHE PATENTSCHRIFT

(11) **EP 2 550 540 B1**
(45) Veröffentlichungstag und Bekanntmachung des Hinweises auf die Patenterteilung: **22.10.2014**
(21) Anmeldenummer: 11714540.9
(22) Anmeldetag: 13.04.2011
(51) Int. Cl.: G01R 33/09, G01R 33/12, G01R 33/00

(54) **VERFAHREN ZUM ERKENNEN VON MAGNETISCH GEKENNZEICHNETEN OBJEKTEN SOWIE ENTSPRECHENDE VORRICHTUNG**
METHOD FOR DETECTING MAGNETICALLY MARKED OBJECTS AND CORRESPONDING DEVICE
PROCÉDÉ POUR DÉTECTER DES OBJETS À MARQUAGE MAGNÉTIQUE AINSI QUE DISPOSITIF CORRESPONDANT

(30) Priorität: 18.05.2010 DE 102010020784
(43) Veröffentlichungstag der Anmeldung: 30.01.2013
(73) Patentinhaber: Siemens Aktiengesellschaft, 80333 München (DE)
(72) Erfinder: ECKERT, Helmut, 91341 Röttenbach (DE)
(86) Internationale Anmeldenummer: PCT/EP2011/055747
(87) Internationale Veröffentlichungsnummer: WO 2011/144391

(56) Entgegenhaltungen:
- US-A1- 2007 269 905
- US-A1- 2009 033 935
- US-A1- 2009 278 534

## Beschreibung

Die Erfindung betrifft ein Verfahren zum insbesondere kontinuierlichen Erkennen von magnetisch gekennzeichneten Objekten, insbesondere biologischen Objekten, vorzugsweise Zellen, eine entsprechende Sensoreinrichtung, eine entsprechende Vorrichtung sowie eine Verwendung.

### Stand der Technik

Derartige Verfahren bzw. Vorrichtungen dienen beispielsweise dazu, Objekte, die ein bestimmtes Merkmal aufweisen, zu identifizieren bzw. zu zählen. Wird beispielsweise eine bestimmte Anzahl von markierten Zellen im Blut eines Patienten gefunden, ist es möglich, eine Krankheit zu diagnostizieren.

Um Zellen detektieren zu können, können diese beispielsweise magnetisch markiert werden, so dass mittels eines magnetoresitiven Sensorelements die magnetisch markierten Zellen erkannt werden können, in dem Änderungen eines externen Magnetfeldes bedingt durch die magnetisch markierten Zellen gemessen werden.

Aus der WO 2008/001261 ist eine magnetische Sensorvorrichtung und ein Verfahren zum Erkennen magnetischer Partikel bekannt geworden. Die magnetische Sensorvorrichtung umfasst dabei Mittel zum Erzeugen eines Magnetfeldes, eine Sensoreinrichtung und eine Kombiniereinrichtung. Die Mittel zum Erzeugen eines Magnetfeldes sind dabei so ausgebildet, dass diese eine Vielzahl von verschiedenen Magnetfeldkonfigurationen erzeugen können. Die Magnetfeldkonfigurationen entsprechen dabei einer Vielzahl von verschiedenen magnetischen Anregungszuständen der magnetischen Partikel.

Aus der US 2008/0024118 A1 ist weiter eine Sensorvorrichtung und ein Verfahren zur Detektion der Anwesenheit von zumindest einem magnetischen Partikel offenbart. Die Sensorvorrichtung umfasst dabei Mittel zur Erzeugung eines magnetischen Feldes und zumindest ein magnetisches Sensorelement. Damit sich die magnetischen Partikel nicht zu dicht an das zumindest eine magnetische Sensorelement annähern und dadurch eine Detektion von ihnen durch das Sensorelement erschwert wird, ist eine Verbotszone zwischen dem magnetischen Sensorelement und dem magnetischen Partikel im Bereich des zumindest einen magnetischen Sensörelementes vorgesehen, die eine Dicke zwischen 1 µm und 300 µm aufweist.

Die US 2007/269905 A1 beschreibt ein Verfahren zum Messen eines Magnetfeldes magnetischer Partikel mit einem Sensorarray.

Die US 2009/033935 A1 beschreibt ein Sensor zur Erkennung von magnetischen Nanopartikeln, bei welchem die magnetischen Nanopartikel mit einem Laser bestrahlt werden und bei welchem die magnetischen Nanopartikel anhand eines Photostroms erkannt werden.

Die US 2009/278534 A1 beschreibt einen Sensor für Magnetpartikel mit einer Anordnung zum Er-zeugen von Magnetfeldern unterschiedlicher Feldkonfigurationen und mit einem Sensor zum Erkennen des Einflusses der Magnetpartikel auf die Magnetfelder.

### Vorteile der Erfindung

Das erfindungsgemäße Verfahren gemäß Anspruch 1, die erfindungsgemäße Vorrichtung gemäß Anspruch 7 sowie die erfindungsgemäße Verwendung gemäß Anspruch 10 weisen den Vorteil auf, dass magnetisch gekennzeichnete Objekte, insbesondere biologische Objekte, vorzugsweise Zellen, trotz einem kleinen Signal-Rausch-Verhältnis beim Erkennen der Objekte einfach und zuverlässig detektiert werden können. Da eine Form des erzeugten Signals auch von äußeren Parametern, beispielsweise Eigenschaften eines Sensors abhängig ist, können derartige äußere Parameter auch für das Aufbereiten des erzeugten Signals genutzt werden, so dass die Erkennung der magnetisch gekennzeichneten Objekte noch weiter verbessert werden kann. Ist das Objekt erkannt, das heißt eine Amplitude des aufbereiteten Signals liegt oberhalb des Schwellwertes, können aus dem Amplitudenwert des erzeugten Signals noch weitere Informationen, beispielsweise über die physikalischen Eigenschaften der Zelle wie beispielsweise Durchmesser, etc. erhalten werden. Daneben ist es auch einfach möglich, beim Auswerten lediglich ein Überschreiten des Schwellwertes der Amplitude des aufbereiteten Signals festzustellen, und so nur die Existenz eines Objekts im Bereich des Sensors festzustellen. PasEigenschaften eines Sensors abhängig ist, können derartige äußere Parameter auch für das Aufbereiten des erzeugten Signals genutzt werden, so dass die Erkennung der magnetisch gekennzeichneten Objekte noch weiter verbessert werden kann. Ist das Objekt erkannt, das heißt eine Amplitude des aufbereiteten Signals liegt oberhalb des Schwellwertes, können aus dem Amplitudenwert des erzeugten Signals noch weitere Informationen, beispielsweise über die physikalischen Eigenschaften der Zelle wie beispielsweise Durchmesser, etc. erhalten werden. Daneben ist es auch einfach möglich, beim Auswerten lediglich ein Überschreiten des Schwellwertes der Amplitude des aufbereiteten Signals festzustellen, und so nur die Existenz eines Objekts im Bereich des Sensors festzustellen. Passieren mehrere Objekte nacheinander den Bereich des Sensors, kann auf diese Weise die Anzahl der Objekte bestimmt werden.

Gemäß einer weiteren vorteilhaften Weiterbildung der Erfindung wird das Magnetfeld im Wesentlichen senkrecht zu einer Richtung der Bewegung der Objekte orientiert, insbesondere, wobei Änderungen des Magnetfeldes, welche bedingt sind durch Änderungen einer magnetischen Flussdichte auf Grund des magnetisch gekennzeichneten Objektes, parallel zur Richtung der Bewegung des Objektes gemessen werden. Der Vorteil hierbei ist, dass damit die Empfindlichkeit eines Sensors für das Erkennen der Objekte möglichst groß ist, da dann eine gemessene Änderung des Magnetfeldes nur von der durch das magnetisch markierte Objekt geänderten magnetischen Flussdichte abhängt. Damit wird auf einfache und zuverlässige Weise ein Erkennen eines Objektes ermöglicht.

Gemäß einer weiteren vorteilhaften Weiterbildung der Erfindung wird die Änderung des Magnetfeldes mittels einer Wheatstone-Brücke gemessen. Der Vorteil hierbei ist, dass durch das Messen mittels einer Wheatstone-Brücke, die im Allgemeinen vier Widerstände umfasst, durch das Vorbeigleiten des magnetisch markierten Objektes über oder an zumindest zwei Widerständen ein anderer Verlauf des erzeugten Signals möglich ist, welches eine genauere Auswertung bzw. Auflösung eines Extremwertes der jeweiligen Objekte und damit ein verbessertes Erkennen der Objekte ermöglicht. Weiterhin ist es ebenfalls möglich, das magnetisch markierte Objekt über mehr als zwei, insbesondere die vier Widerstände der Wheatstone-Brücke passieren zu lassen. Die Widerstände können dann so angeordnet werden, dass die Zelle groß gegenüber der räumlichen Ausdehnung der vier Widerstände ist, mithin also beim Vorbeigleiten des Objektes an den vier Widerständen ein einzelnes Signal erzeugt wird, welches jedoch die entsprechende vielfache Amplitude eines Signals eines einzelnen Widerstandes aufweist.

Gemäß einer weiteren vorteilhaften Weiterbildung der Erfindung umfasst das Aufbereiten des erzeugten Signals ein Glätten, insbesondere durch Falten des erzeugten Signals mit einer Gauß-Funktion. Der Vorteil hierbei ist, dass damit hochfrequente Anteile des erzeugten Signals eliminiert werden können, was letztlich ein Auswerten des erzeugten Signals verbessert. Das Aufbereiten des erzeugten Signals kann dabei ebenfalls eine Tiefpassfilterung umfassen. Der Vorteil hierbei ist, dass ein Signal-Rausch-Verhältnis des erzeugten Signals verbessert wird, um eine verbesserte Erkennung eines magnetisch markierten Objektes anhand der gemessenen Änderungen des Magnetfeldes zu erreichen.

Gemäß einer weiteren vorteilhaften Weiterbildung der Erfindung umfasst das Aufbereiten und/oder Auswerten des erzeugten Signals ein Falten des Signals mit zumindest einer Ableitung der Gauß-Funktion der Ordnung größer oder gleich 1. Der Vorteil hierbei ist, dass das Ermitteln von Extremwerten anhand des erzeugten und/oder aufbereiteten Signals durch das Falten mit einer n-ten Ableitung einer Gauß-Funktion mit n ≥ 1, wobei n eine natürliche Zahl ist, erheblich verbessert wird, insbesondere bei n = 2. Durch das Falten werden Bereiche des erzeugten Signals mit großer Veränderung hinsichtlich ihrer Steigung hervorgehoben, das heißt, angewandt auf das erzeugte Signal werden Bereiche des Signals hervorgehoben, welche eine schnelle bzw. starke Änderung der Amplitude des erzeugten Signals aufweisen. Damit wird dann eine Auswertung des Signals bzw. das Ermitteln von Extremwerten des Signals vereinfacht.

Gemäß einer weiteren vorteilhaften Weiterbildung der Erfindung wird das Aufbereiten des erzeugten Signals anhand einer Geschwindigkeit der Objekte und/oder von Abmessungen einer Sensoreinrichtung vorgenommen. Der Vorteil hierbei ist, dass dies bekannte externe Größen sind, und diese während der Durchführung des Verfahrens im Wesentlichen konstant und/oder bekannt bleiben. Diese Größen können dann beim Aufbereiten und Auswerten des Signals herangezogen werden, so dass insgesamt die Genauigkeit des Verfahrens gesteigert wird.

Gemäß einer weiteren vorteilhaften Weiterbildung der Erfindung wird der Schwellwert dynamisch angepasst, insbesondere durch statistische Methoden. Der Vorteil hierbei ist, dass keine Probemessungen vorab durchgeführt werden müssen, um einen Schwellwert festzulegen. Die Durchführung des Verfahrens wird damit erheblich vereinfacht und die Zeit für ein Erkennen für eine bestimmte Anzahl von magnetisch markierten Objekten wird verkürzt.

Weitere Beispiele der Erfindung sind in der Zeichnung dargestellt und in der nachfolgenden Beschreibung näher erläutert. Es zeigen
- Fig. 1a: ein Diagramm eines Verlaufs einer gemessenen Widerstandsänderung eines Sensors beim Vorbeigleiten einer magnetisch markierten Zelle in Abhängigkeit der Zeit;
- Fig. 1b-d: Prinzipschaubilder einer an einem Sensor vorbeigleitenden Zelle;
- Fig. 2: ein Prinzipschaubild einer Sensoreinrichtung mit einem Sensor in Form einer Wheatstone-Brücke;
- Fig. 3a: ein Diagramm eines Verlaufs einer gemessenen Änderung einer Brückenspannung einer Wheatstone-Brücke beim Vorbeigleiten einer magnetisch markierten Zelle in Abhängigkeit der Zeit;
- Fig. 3b-d: Prinzipschaubilder einer an einem Sensor gemäß Figur 2 vorbeigleitenden Zelle;
- Fig. 4a-d: zeitliche Verläufe der Amplituden von Signalen eines Sensors nach Durchführung von verschiedenen Verfahrensschritten;
- Fig. 5: ein Prinzipschaubild des Ablaufs eines Verfahrens gemäß einer ersten Ausführungsform der Erfindung; sowie
- Fig. 6: ein Prinzipschaubild einer weiteren Ausführungsform einer Sensoreinrichtung mit einem Sensor in Form einer Wheatstone-Brücke;

Soweit nicht anders in der Beschreibung zu den Figuren beschrieben, beziehen sich gleiche Bezugszeichen auf dieselben bzw. funktionsgleiche Elemente.

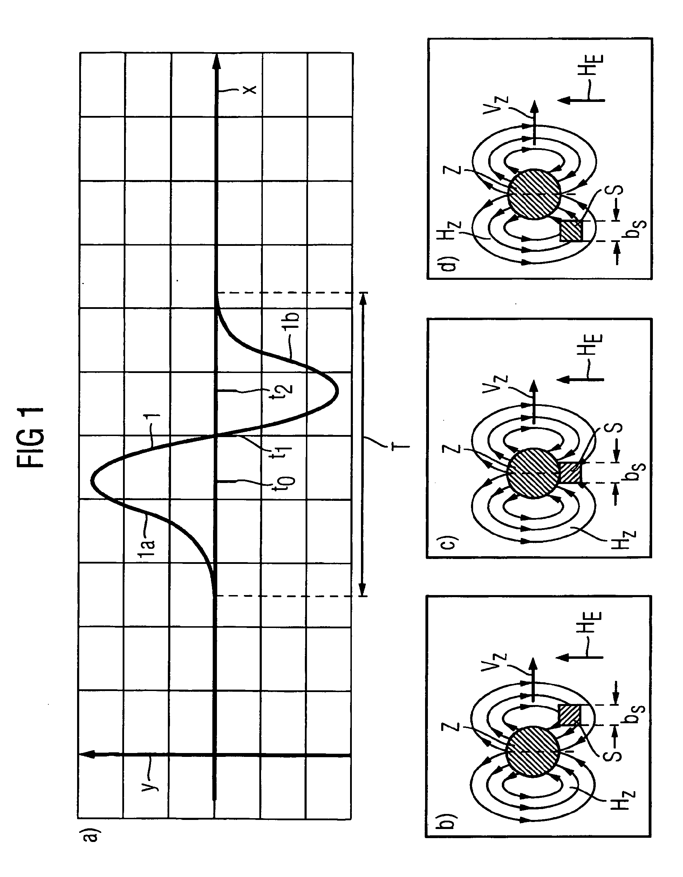

In den Figuren 1-6 ist jeweils zum besseren Verständnis der Erfindung und den jeweiligen Ausführungsformen vereinfachend ein Magnetfeld H_{Z} einer magnetisch markierten Zelle Z explizit gezeigt. Eine reale magnetisch markierte Zelle Z weist im Allgemeinen jedoch kein eigenes Magnetfeld H_{Z} auf, sie ist lediglich magnetisch markiert, beispielsweise mit magnetisierbaren Stoffen, insbesondere mit weich- oder ferromagnetischen Partikeln. Diese Partikel erzeugen, eingebracht zusammen mit der Zelle Z in einem Magnetfeld H_{E} eine lokale Magnetfeldflussänderung, die eine Änderung des Magnetfeldes H_{E} im Bereich der Position der Zelle Z hervorruft, welche beim Vorbeigleiten der Zelle Z an einem entsprechend ausgebildeten Sensor detektiert werden kann.

Das in den Figuren 1-6 gezeigte Magnetfeld H_{Z} der Zelle Z bewirkt ebenfalls eine lokale Änderung des Magnetfeldes H_{E} im Bereich der Position der Zelle Z. Wie vorstehend beschrieben kann diese dann mit einem entsprechend ausgebildeten Sensor detektiert werden. In den Figuren 1-6 ist somit eine Zelle Z mit einem Magnetfeld H_{Z} als magnetisch markierte Zelle Z zu verstehen, die in einem Magnetfeld H_{E} eine lokale Änderung des Magnetfeldes H_{E} im Bereich Ihrer Position erzeugt.

Fig. 1a zeigt ein Diagramm eines Verlaufs einer gemessenen Widerstandsänderung eines Sensors beim Vorbeigleiten einer magnetisch markierten Zelle in Abhängigkeit der Zeit.

In Fig. 1a ist auf der x-Achse X die Zeit aufgetragen und auf der y-Achse Y ist eine Widerstandsänderung eines Sensors in Form eines magnetoresistiven Elementes S aufgetragen. Nähert sich nun die magnetisch markierte Zelle Z mit Magnetfeld H_{Z} gemäß Fig. 1b dem magnetoresistiven Element bzw. Sensor S mit einer Geschwindigkeit v_{Z} und ist ein externes Magnetfeld H_{E} senkrecht zur Bewegungsrichtung der Zelle Z vorhanden, erzeugt der magnetoresistive Sensor S ein Signal mit dem zeitlichen Verlauf 1 der Widerstandsänderung gemäß Fig. 1a, wenn dieser eine lokale Änderung des Magnetfeldes H_{E} auf Grund des Magnetfeldes H_{Z} der Zelle Z detektiert: Ist die Zelle Z noch weit vom Sensor S entfernt, erfährt der Sensor S keine Widerstandsänderung, das heißt die Kurve 1 verläuft auf der x-Achse bzw. die Widerstandsänderung ist Null.

Nähert sich die magnetisch markierte Zelle Z dem Sensor S von links nach rechts, erfährt der Sensor S eine Widerstandsänderung durch das Magnetfeld H_{Z}, genauer der Komponente des Magnetfeldes H_{Z}, die parallel zur Bewegungsrichtung der Zelle Z orientiert ist und die Kurve 1 steigt an (Verlauf 1a gemäß Fig. 1a). Bewegt sich die Zelle Z nun weiter gemäß Fig. 1b von links nach rechts in Richtung des Sensors S fällt die Kurve 1 ab und erreicht zu einem Zeitpunkt t₁ > t₀ wiederum Null. Zum Zeitpunkt t₁ befindet sich die Zelle Z mit kleinstmöglichem Abstand zum Sensor S. Da die Achse des Magnetfeldes H_{Z} der Zelle Z und eine Mittelachse des Sensors S deckungsgleich sind misst der Sensor S kein Magnetfeld H_{Z} der Zelle Z und damit auch keine Widerstandsänderung, da dieser nur sensitiv in Richtung der Bewegungsrichtung der Zelle Z ausgebildet ist; die Widerstandsänderung ist Null.

Bewegt sich die Zelle Z nun weiter von links nach rechts gemäß Fig. 1c bzw. Fig. 1d misst der Sensor S nun eine negative Widerstandsänderung 1b, da die Feldlinien des Magnetfeldes H_{Z} der Zelle Z nun in die entgegengesetzte Richtung im Bereich des Sensors S orientiert sind. Entfernt sich die Zelle Z mit der Geschwindigkeit v_{Z} weiter von dem Sensor S nimmt die negative Widerstandsänderung 1b wieder ab, so dass in genügender Entfernung der Zelle Z vom Sensor S keine Widerstandsänderung mehr detektiert wird, der Verlauf 1 also wiederum Null ist.

Insgesamt ist der Verlauf 1 punktsymmetrisch zum Zeitpunkt t₁ ausgebildet und weist zu den Zeitpunkten t₀ und t₂ Extremwerte der Widerstandsänderung auf. Als Periodendauer T wird im Wesentlichen die Zeitspanne ab dem von Null ansteigenden Verlauf 1a mit Extremwert zum Zeitpunkt t₀, der Nulldurchgang t₁, das zweite Extremum der negativen Widerstandsänderung 1b zum Zeitpunkt t₂ bis zum erneuten im Wesentlichen konstanten Verlauf der Widerstandsänderung gleich Null definiert.

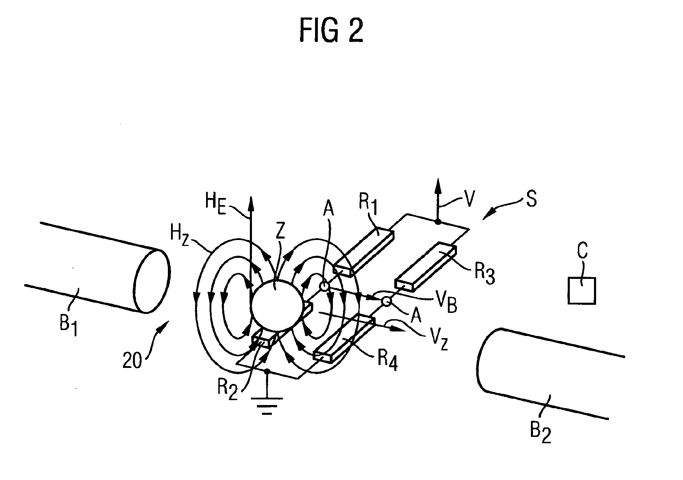

Fig. 2 zeigt ein Prinzipschaubild einer Sensoreinrichtung mit einem Sensor in Form einer Wheatstone-Brücke.

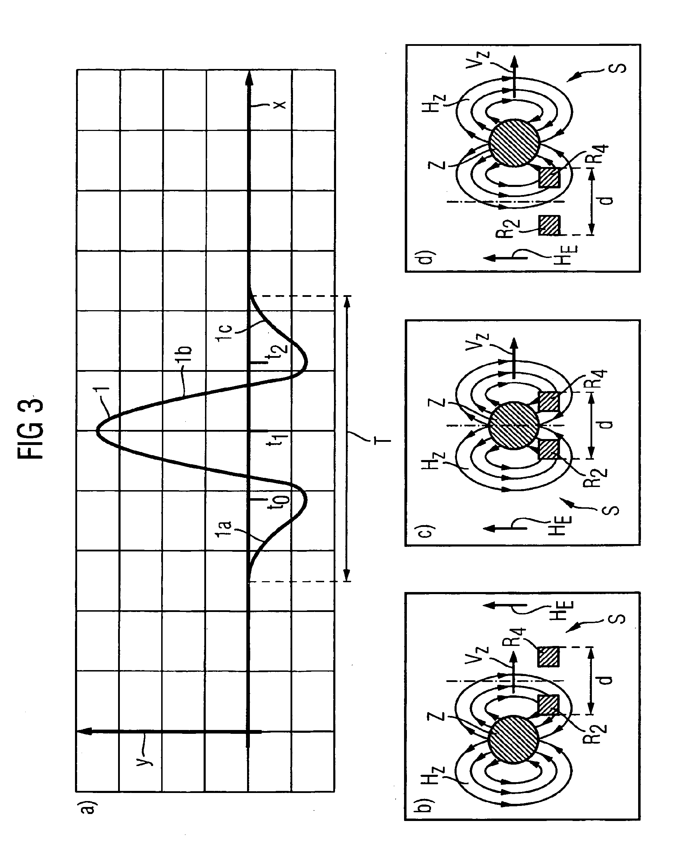

In Fig. 2 ist eine perspektivische Ansicht eines Prinzipschaubildes einer Wheatstone-Messbrücke mit Widerständen R₁-R₄ gezeigt. Die Zelle Z ist dabei wiederum magnetisch gekennzeichnet, das heißt sie weist ein Magnetfeld H_{Z} auf. Die Zelle Z bewegt sich nun mit der Geschwindigkeit v_{Z} nacheinander über zwei Widerstände R₂, R₄ der Wheatstone-Messbrücke R₁-R₄ gemäß Fig. 3b-d und erzeugt dabei eine Änderung einer Brückenspannung V_{B}, die zwischen den Widerständen R₁, R₂, R₃, R₄ entsprechend dem Prinzip einer Wheatstone-Messbrücke anliegt. Das externe Magnetfeld H_{E} ist dabei senkrecht zu der Richtung der Geschwindigkeit v_{Z} der Zelle Z orientiert.

Weiterhin sind in Figur 2 dünne Röhrchen B₁, B₂ angedeutet zu sehen, die zum Zuführen der Zelle Z zum Sensor S in Form der Wheatstone-Messbrücke mit Widerständen R₁, R₂, R₃, R₄ und auch zum Entfernen der Zelle Z dienen. Selbstverständlich ist auch jeder andere mögliche Art von Zuführ- oder Entfernmitteln möglich. Ebenfalls ist eine Auswerteeinheit in Form eines Computers C gezeigt, die zum Auswerten eines aufbereiteten Signals dient. Der Computer C übernimmt dabei auch das Aufbereiten F₁, F₂, F₃, F₄ eines Signals V_{B} des Sensors S und ist hierfür mit Anschlüssen A zum Abgreifen der Brückenspannung V_{B} der Wheatstone-Messbrücke verbunden (nicht gezeigt).

Fig. 3a zeigt ein Diagramm eines Verlaufs einer gemessenen Änderung einer Brückenspannung einer Wheatstone-Brücke beim Vorbeigleiten einer magnetisch markierten Zelle in Abhängigkeit der Zeit.

In Fig. 3a ist nun der Verlauf der Änderung einer Brückenspannung V_{B} gezeigt, wobei auf der y-Achse Y die Änderung der Brückenspannung V_{B} und auf der x-Achse X die Zeit aufgetragen ist. Die Kurve 1 zeigt den Verlauf der Änderung der Brückenspannung V_{B} beim Vorbeigleiten der Zelle Z an den Widerständen R₂, R₄ der Wheatstone-Messbrücke R₁, R₂, R₃, R₄. Dieser ist dabei wie folgt:
Bewegt sich die Zelle Z mit ihrem Magnetfeld H_{Z} auf die beiden Widerstände R₂, R₄ gemäß Fig. 3b und analog wie in den Figuren 1b-1d von links nach rechts mit der Geschwindigkeit v_{Z} zu, wird zunächst der Widerstand R₂ vom Magnetfeld H_{Z} der Zelle Z beaufschlagt. Dabei wird eine negative Brückenspannung V_{B} mit Verlauf 1a erzeugt, die einen Extremwert zum Zeitpunkt t₀ gemäß der Fig. 3a aufweist. Bewegt sich die Zelle Z weiter von links nach rechts gemäß Figur 3c schwächt sich die negative Änderung der Brückenspannung V_{B} wieder ab und steigt dann im weiteren zeitlichen Verlauf bis auf einen positiven Extremwert zum Zeitpunkt t₁ an. Zum Zeitpunkt t₁ befindet sich nun die Zelle Z zwischen den beiden Widerständen R₂ und R₄, die im Abstand d voneinander beabstandet sind, das heißt die Zelle Z befindet sich im Wesentlichen in der Mitte zwischen den beiden Widerständen R₂ und R₄.

Bewegt sich nun die Zelle Z weiter von links nach rechts gemäß Figur 3d, so schwächt sich der weitere Verlauf 1b der positiven Änderung der Brückenspannung V_{B} wieder ab, durchläuft eine Nullstelle und wird im weiteren zeitlichen Verlauf 1c wieder negativ. Der Verlauf 1c weist wiederum zum Zeitpunkt t₂ einen Extremwert auf. Zum Zeitpunkt t₂ wird nur (noch) der Widerstand R₄ vom Magnetfeld H_{Z} der Zelle Z beaufschlagt, analog zum Widerstand R₂ gemäß Fig. 3b.

Insgesamt ist somit die Kurve 1 der Änderung der Brückenspannung V_{B} spiegelsymmetrisch zum Zeitpunkt t₁. Die Periodendauer T wird entsprechend der Beschreibung zu Fig. 1 definiert, nämlich als die Zeitdauer von der ersten Änderung der Brückenspannung V_{B}, die von Null verschieden ist bis die Änderung der Brückenspannung V_{B} wiederum Null ist und die Zelle Z an den beiden Widerständen R₂, R₄ vorbeigeglitten ist.

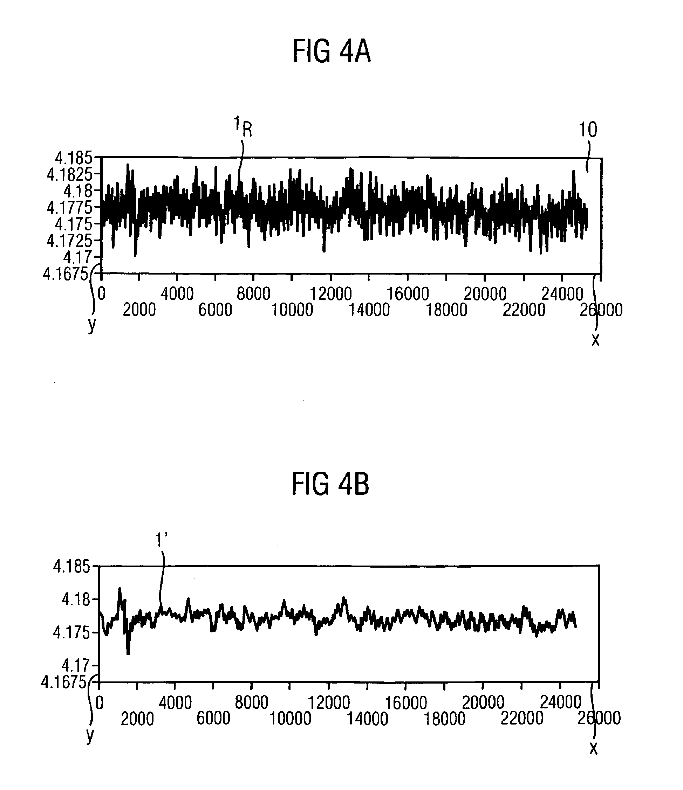

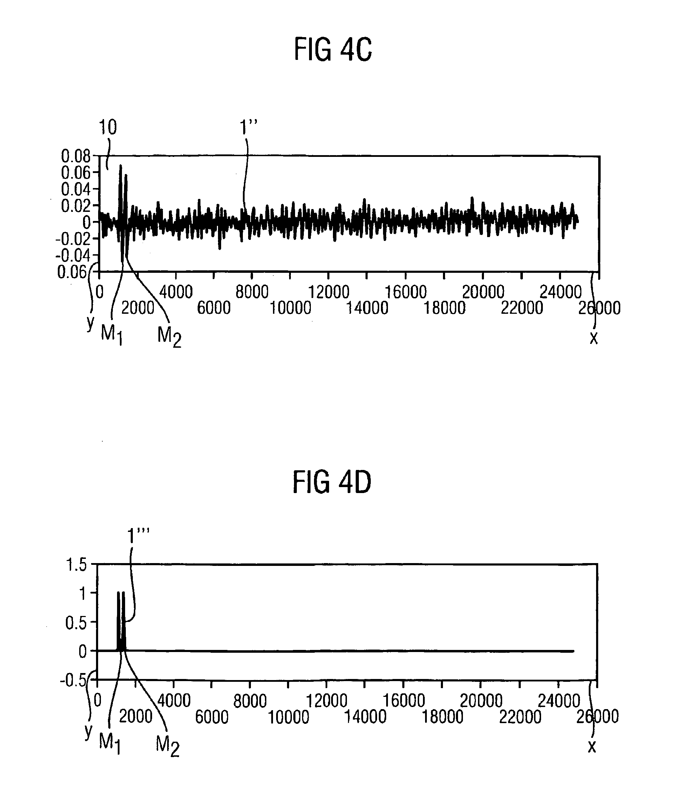

Fig. 4 zeigt zeitliche Verläufe der Amplituden von Signalen eines Sensors nach Durchführung von verschiedenen Verfahrensschritten.

In Fig. 4a ist ein x-y-Diagramm gezeigt, wobei die x-Achse eine Zeitachse ist und die y-Achse Y eine positive Amplitude eines Signals 1_{R} darstellt, erzeugt von einem Sensor S gemäß den Fig. 1b-1d. An dem Sensor S sind dabei zwischen den Zeitpunkten t=0 und t=2000 zwei magnetisch markierte Zellen Z vorbeigeglitten bzw. sie bewegten sich an diesem vorbei. Weiterhin ist ein Schwellwert 10 bei einer Amplitude von +4,1825 festgelegt. Zu erkennen sind mehrere Spitzen der Amplitude des Signals 1_{R}, die den Schwellwert 10 überschreiten. Diese sind nicht nur im Bereich zwischen t=0 und t=2000 zu sehen, sondern auch zu den Zeitpunkten t>2000, bei denen keine Zelle Z den Sensor S passiert hat. Zur Aufbereitung und Auswertung des Signals 1_{R} wird dieses nun digitalisiert bzw. in ein zeitdiskretes Signal 1 umgewandelt. Dabei muss in Abhängigkeit von der Geschwindigkeit v_{z}, mit der eine Zelle Z an dem Sensor S vorbei gleitet bzw. diesen passiert und der daraus resultierenden Periodendauer T gemäß Abbildungen Fig. 3a bzw. 1a das Nyquist-Shannon-Abtasttheorem eingehalten werden.

Im Anschluss daran wird das digitalisierte Signal 1 geglättet, um hochfrequente Anteile zu eliminieren. Hierzu wird das digitalisierte Signal 1 gemäß Fig. 4a mit einer Gauß-Funktion gefaltet, wobei das so geglättete Signal 1' in Fig. 4b dargestellt ist. Dieses geglättete Signal 1' wird nun mit einer zweiten partiellen Ableitung einer Gauß-Funktion gefaltet. Aufgrund der vorangegangenen Diskretisierung des Signals 1' berechnet sich der Verlauf 1'' des gefalteten Signals anhand einer diskreten Summe eines Produkts aus geglättetem Signal 1' und zweiter Ableitung der Gauß-Funktion. Der Summationsindex der diskreten Summe ist dabei von Parametern, das heißt externen, bekannten Größen abhängig, die zur Optimierung der Glättung bzw. einer weiteren Extremwertfilterung dienen. Diese umfassen unter anderem eine laminare Strömungsgeschwindigkeit bzw. die Geschwindigkeit v_{Z}, mit der sich die Zelle Z bewegt. Ist der Sensor S als Wheatstone-Brücke R₁, R₂, R₃, R₄ ausgebildet, kann als weiterer Parameter der jeweilige Abstand d der Widerstände R₂, R₄ parallel zur Bewegungsrichtung der Zelle Z verwendet werden. Ist ein einzelner Widerstand R vorhanden, kann dessen Breite b verwendet werden.

In Fig. 4c ist nun ein diskretisiertes, geglättetes und mit einer zweiten Ableitung einer Gauß-Funktion gefaltetes Signal 1'' in dem entsprechenden Verlauf gemäß den Fig. 4a bzw. 4b gezeigt. Zu erkennen sind nun lokale Maxima M₁, M₂, die einem Vorbeigleiten einer Zelle Z an dem Sensor S entsprechen. Diese lokalen Maxima M₁, M₂ heben sich von dem weiteren Verlauf des Signals 1'' hinsichtlich ihrer Amplitude deutlich ab. Um nun zu entscheiden, ob eine Zelle Z erkannt wird oder nicht, wird ein statischer Schwellenwert 10 festgelegt. In Fig. 4c ist dieser 0,04. Es werden also lediglich die Amplituden des Signals 1'' gemäß Fig. 4c ausgewertet, deren Amplitude größer ist als 0,04. Fig. 4d zeigt eine Amplitude des Signals 14 nach Durchführung einer Filterung mit dem vorstehend genannten Schwellwert 10. Es sind lediglich noch zwei Werte der Größe 1 zu sehen, die den Maxima M₁ bzw. M₂ der Fig. 4c entsprechen. Das Filtern nach dem Schwellwert 10 liefert also bei Vorbeigleiten einer Zelle Z an dem Sensor S eine logische 1 wie in der Fig. 4d gezeigt.

Aus dem jeweiligen Amplitudenwert gemäß Fig. 4a, an den Maxima M₁, M₂ oberhalb des Schwellwertes 10 können auch zusätzliche Informationen über physikalische Eigenschaften der Zelle Z, wie beispielsweise Durchmesser der Zelle Z, etc. erhalten werden. Mittels dieser Schwellwertfilterung können gemäß Fig. 4d auch lediglich die Anzahl der den Sensor S passierten Zellen Z gemessen werden. Daneben ist es möglich, die Zuverlässigkeit der Schwellwertfilterung weiter zu verbessern, indem beispielsweise das Signal 1'' gemäß Fig. 4c erneut geglättet wird.

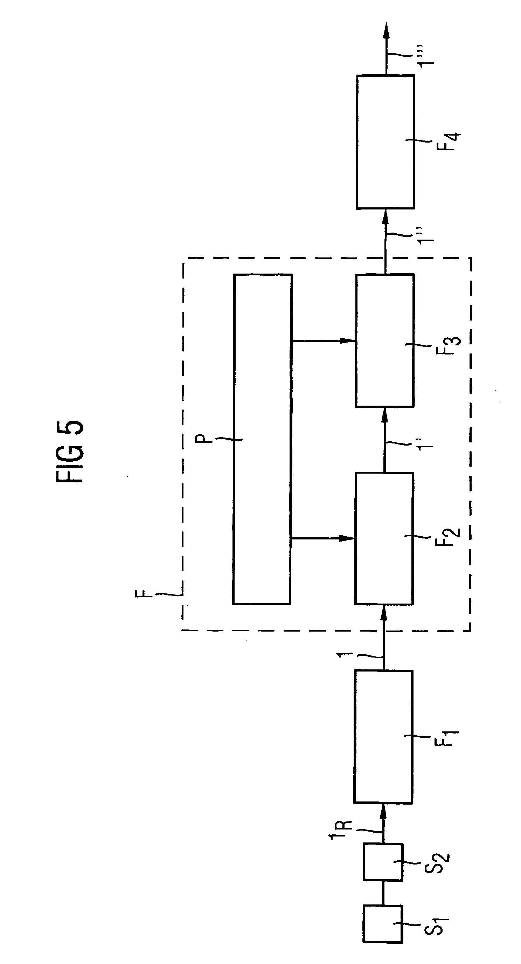

Fig. 5 zeigt ein Prinzipschaubild des Ablaufs eines Verfahrens gemäß einer ersten Ausführungsform der Erfindung.

In Fig. 5 ist ein erfindungsgemäßes Verfahren gemäß der ersten Ausführungsform gezeigt. Wird das magnetisch markierte Objekt Z in einem ersten Schritt S₁ in einem Magnetfeld H_{E} bewegt, wird weiter eine durch das Objekt Z bedingte lokale Änderung des Magnetfeldes H_{E} aufgrund seiner Bewegung vorbei an einem Sensor S in einem zweiten Schritt S₂ gemessen. Das vom Sensor S erzeugte Signal 1_{R} wird zunächst in einem Analog-Digital-Wandler F₁ digitalisiert und in ein zeitdiskretes Signal 1 gewandelt. Das digitalisierte Signal 1 wird dann mittels eines Filters F₂ geglättet, das geglättete Signal 1' wird in einem weiteren Filter F₃ zur Bestimmung der Extremwerte mittels Faltung des Signals 1' mit einer zweiten Ableitung einer Gauß-Funktion aufbereitet. Das resultierende Signal 1'' wird dann anhand eines Schwellwertes in einem Schwellwertfilter F₄ gefiltert.

Das vom Schwellwertfilter 4 ausgegebene Signal 1''' entspricht dann beispielsweise dem Verlauf gemäß Fig. 4d. Das vom Extremwertfilter F₃ ausgegebene Signal 1'' entspricht dem Signal 1'' gemäß Fig. 4c, das Signal 1', ausgegeben vom Filter F₂, weist dabei den Verlauf gemäß Fig. 4b auf. Das Signal 1 gemäß Fig. 4a entspricht dabei dem Signal 1 ausgegeben von dem Analog-Digital-Wandler F₁. Die Parameter P für einen Filter F, umfassend die Filter F₂ und F₃, entsprechen externen bekannten Größen beispielsweise der laminaren Strömungsgeschwindigkeit bzw. der Geschwindigkeit v_{Z}, mit der sich ein Objekt Z einem Sensor S vorbeibewegt, einer Breite b des Sensors S bzw. einem Abstand d von Widerständen R₁, R₂, R₃, R₄ einer Wheatstone-Messbrücke.

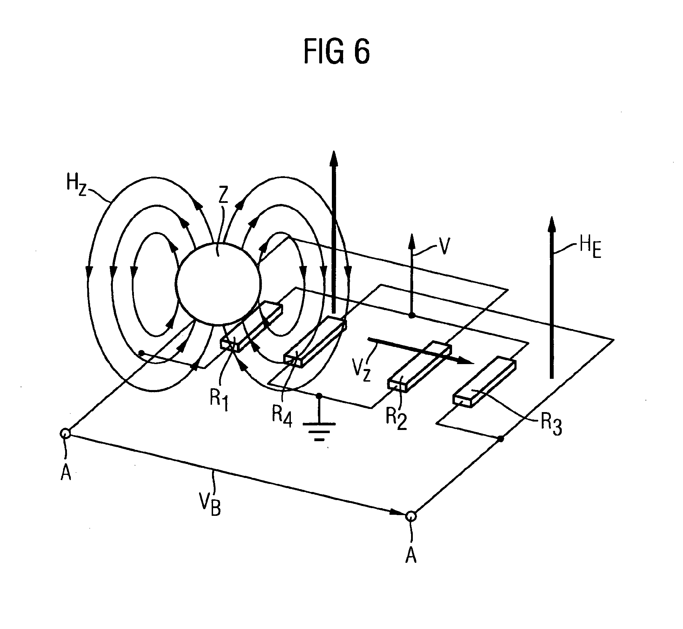

Fig. 6 zeigt ein Prinzipschaubild einer weiteren Ausführungsform einer Sensoreinrichtung mit einem Sensor in Form einer Wheatstone-Brücke.

In Fig. 6 ist schließlich eine perspektivische Ansicht eines Prinzipschaubildes einer Wheatstone-Messbrücke mit Widerständen R₁-R₄ gezeigt. Die Zelle Z ist dabei wiederum magnetisch gekennzeichnet. Die Zelle Z bewegt sich nun mit der Geschwindigkeit v_{Z} nacheinander über vier Widerstände R₁, R₄, R₂, R₃ der Wheatstone-Messbrücke R₁-R₄ analog zu den Fig. 3b-d und erzeugt dabei eine Änderung einer Brückenspannung V_{B}, die zwischen den Widerständen R₁, R₂, R₃, R₄ entsprechend dem Prinzip einer Wheatstone-Messbrücke anliegt. Das externe Magnetfeld H_{E} ist dabei senkrecht zu der Richtung der Geschwindigkeit v_{Z} der Zelle Z orientiert. Die Änderung der Brückenspannung V_{B} weist dabei im Wesentlichen denselben Verlauf auf gemäß der Figur 3a. Der gegebenenfalls als Parameter für das Filtern relevante Abstand d gemäß Figur 3b-3d, ist dabei nicht der Abstand zwischen den beiden Widerständen R₂, R₄, sondern der Abstand zwischen dem Mittelpunkt des Abstandes zwischen den Widerständen R₁, R₄ und dem Mittelpunkt des Abstandes zwischen den Widerständen R₂, R₃ entlang der Bewegungsrichtung der Zelle Z.

Der weitere Aufbau der Vorrichtung gemäß Figur 6, also dünne Röhrchen, etc. entspricht dem der Figur 2.

Obwohl die vorliegende Erfindung anhand vorstehender Ausführungsbeispiele beschrieben wurde, ist sie nicht darauf beschränkt, sondern auf vielfältige Weise modifizierbar.

## Patentansprüche

1. Verfahren zum insbesondere kontinuierlichen Erkennen von magnetisch gekennzeichneten Objekten (Z), insbesondere biologischen Objekten, vorzugsweise Zellen, umfassend die Schritte
Bewegen (S₁) zumindest eines magnetisch gekennzeichneten Objektes (Z) in einem Magnetfeld (H_{E}),
Messen (S₂) einer lokalen Änderung des Magnetfeldes (H_{E}) bedingt durch das magnetisch gekennzeichnete Objekt (Z), Erzeugen (F₁) eines insbesondere digitalisierten Signals (1) anhand der gemessenen lokalen Änderung des Magnetfeldes (H_{E}),
Aufbereiten (F₂, F₃) des erzeugten Signals (1) mittels zumindest einem Falten des erzeugten Signals (1) mit einer mathematischen Funktion,
Auswerten (F₄) des aufbereiteten Signals (1''), wobei das Auswerten des Signals (1'') das Ermitteln (F₄) von Extremwerten (M₁, M₂), insbesondere Maximalwerten, des Signals (1'') und Vergleichen der ermittelten Extremwerte (M₁, M₂) mit einem insbesondere vorgegebenen Schwellwert (10) umfasst, bei dessen Überschreiten das Objekt (Z) als erkannt gilt,
**dadurch gekennzeichnet,**
**dass** das Aufbereiten des erzeugten Signals (1) ein Glätten (F₂) durch Falten des erzeugten Signals (1) mit einer Gauss-Funktion umfasst.

2. Verfahren gemäß Anspruch 1, wobei
das Magnetfeld (H_{E}) im Wesentlichen senkrecht zu einer Richtung der Bewegung des Objektes (Z) orientiert wird, insbesondere wobei Änderungen des Magnetfeldes (H_{E}), welche bedingt sind durch Änderungen einer magnetischen Flussdichte auf Grund des magnetisch gekennzeichneten Objektes (Z), parallel zur Richtung der Bewegung des Objektes (Z) gemessen werden.

3. Verfahren gemäß zumindest Anspruch 1, wobei die Änderung des Magnetfeldes (H_{E}) mittels einer Wheatstone-Brücke (R₁, R₂, R₃, R₄) gemessen wird.

4. Verfahren gemäß zumindest Anspruch 1, wobei das Aufbereiten (F₂, F₃) und/oder Auswerten (F₄) des erzeugten Signals (1) ein Falten (F₃) des Signals (1) mit zumindest einer Ableitung der Gauss-Funktion der Ordnung größer oder gleich Eins umfasst.

5. Verfahren gemäß zumindest Anspruch 1, wobei das Aufbereiten (F₂, F₃) des erzeugten Signals (1) anhand einer Geschwindigkeit (v_{Z}) der Objekte (Z) und/oder von Abmessungen eines Sensors (b, d) vorgenommen wird.

6. Verfahren gemäß zumindest Anspruch 1, wobei der Schwellwert (10) dynamisch angepasst wird, insbesondere durch statistische Methoden.

7. Vorrichtung zum insbesondere kontinuierlichen Erkennen von magnetisch gekennzeichneten Objekten (Z), insbesondere biologischen Objekten, vorzugsweise Zellen, und insbesondere geeignet zur Ausführung eines Verfahrens gemäß zumindest einem der Ansprüche 1-6, umfassend eine Sensoreinrichtung (20) zum insbesondere kontinuierlichen Erkennen von magnetisch gekennzeichneten Objekten,
Mittel (B₁) zum Zuführen zumindest eines Objektes (Z) hin zur Sensoreinrichtung (20),
Mittel (B₂) zum Entfernen des zumindest einen Objektes (Z) von der Sensoreinrichtung (20),
Mittel (F₁, F₂, F₃) zum Erzeugen und Aufbereiten eines von der Sensoreinrichtung (20) erzeugten Signals (1, V_{B}), ausgebildet zum zumindest einmaligen Falten des erzeugten Signals (1, V_{B}) mit einer Gauss-Funktion,
eine Auswerteeinheit (C), die das aufbereitete Signal (1'') auswertet, derart, dass das Auswerten des Signals (1'') das Ermitteln (F₄) von Extremwerten (M₁, M₂) des Signals (1'') und Vergleichen der ermittelten Extremwerte (M₁, M₂) mit einem insbesondere vorgegebenen Schwellwert (10) umfasst, bei dessen Überschreiten das Objekt (Z) als erkannt gilt.

8. Vorrichtung nach Anspruch 7,
wobei die Sensoreinrichtung (20) aufweist:
Mittel zum Erzeugen eines Magnetfeldes (HE), einen Sensor (S) zur Messung eines Magnetfeldes, umfassend insbesondere zumindest einen elektrischen Widerstand (R, R1, R2, R3, R4) wobei
der Sensor (S) senkrecht zu einer Richtung des erzeugten Magnetfeldes (HE) orientiert ist und der Sensor (S) lokale Änderungen des Magnetfeldes (HE) bedingt durch das magnetisch gekennzeichnete Objekt (Z) parallel zu einer Bewegungsrichtung des Objektes (Z) misst, bei einem Bewegen des Objektes (Z) im Bereich des Sensors (S),
Mittel (A) zum Bereitstellen eines Signals (VB, 1) des Sensors (S).

9. Vorrichtung nach Anspruch 8, wobei
der Sensor (S) als Wheatstone-Messbrücke (R, R1, R2, R3, R4) ausgebildet ist.

10. Verwendung von Extremwerten (M₁, M₂) eines Signals (1, 1', 1'', 1''') eines Sensors (S) zur Messung eines Magnetfeldes beim Bewegen von magnetisch gekennzeichneten Objekten (Z) in einem Magnetfeld (H_{E}) zum, insbesondere kontinuierlichen, Erkennen von den magnetisch gekennzeichneten Objekten (Z) im Bereich des Sensors (S) anhand eines Schwellwertes (10) nach einem Aufbereiten (F₂, F₃) eines vom Sensor (S) erzeugten Signals (1) mittels zumindest einer Faltung des erzeugten Signals (1) mit einer n-ten Ableitung einer Gauss-Funktion mit n≥0, wobei n eine natürliche Zahl inklusive der Zahl Null ist.

## Claims

1. Method for detecting, in particular continuously detecting, magnetically marked objects (Z), in particular biological objects, preferably cells, comprising the steps of
moving (S₁) at least one magnetically marked object (Z) in a magnetic field (H_{E}),
measuring (S₂) a local change in the magnetic field (H_{E}) caused by the magnetically marked object (Z), generating (F₁) a signal (1), in particular a digitised signal (1), on the basis of the measured local change in the magnetic field (H_{E}),
conditioning (F₂, F₃) the generated signal (1) by means of at least one convolution of the generated signal (1) using a mathematical function,
evaluating (F₄) the conditioned signal (1''), wherein the evaluation of the signal (1'') comprises determining (F₄) extreme values (M₁, M₂), in particular maximum values, of the signal (1'') and comparing the determined extreme values (M₁, M₂) with a threshold value (10), in particular a predefined threshold value (10), which, if exceeded, indicates detection of the object (Z),
**characterised in that**
the conditioning of the generated signal (1) comprises a smoothing (F₂) by means of convolution of the generated signal (1) using a Gaussian function.

2. Method according to claim 1, wherein
the magnetic field (H_{E}) is oriented substantially vertically with respect to a direction of movement of the object (Z), in particular wherein changes in the magnetic field (H_{E}) which are caused by changes in a magnetic flux density on account of the magnetically marked object (Z) are measured in parallel with the direction of movement of the object (Z).

3. Method according to at least claim 1, wherein
the change in the magnetic field (H_{E}) is measured by means of a Wheatstone bridge (R₁, R₂, R₃, R₄).

4. Method according to least claim 1, wherein
the conditioning (F₂, F₃) and/or evaluation (F₄) of the generated signal (1) comprises a convolution (F₃) of the signal (1) using at least one derivative of the Gaussian function of the order of greater than or equal to one.

5. Method according to at least claim 1, wherein
the conditioning (F₂, F₃) of the generated signal (1) is performed on the basis of a velocity (v_{Z}) of the objects (Z) and/or on dimensions of a sensor (b, d).

6. Method according to at least claim 1, wherein
the threshold value (10) is adjusted dynamically, in particular by means of statistical methods.

7. Device for detecting, in particular continuously detecting, magnetically marked objects (Z), in particular biological objects, preferably cells, and in particular suitable for performing a method as claimed in at least one of claims 1-6, comprising
a sensor device (20) for detecting, in particular continuously detecting, magnetically marked objects, means (B₁) for supplying at least one object (Z) to the sensor device (20),
means (B₂) for removing the at least one object (Z) from the sensor device (20),
means (F₁, F₂, F₃) for generating and conditioning a signal (1, V_{B}) generated by the sensor device (20), embodied for at least one-time convolution of the generated signal (1, V_{B}) using a Gaussian function,
an evaluation unit (C) which evaluates the conditioned signal (1'') in such a way that the evaluation of the signal (1'') comprises determining (F₄) extreme values (M₁, M₂) of the signal (1'') and comparing the determined extreme values (M₁, M₂) with a threshold value (10), in particular a predefined threshold value (10), which, if exceeded, indicates detection of the object (Z).

8. Device according to claim 7,
wherein the sensor device (20) has:
means for generating a magnetic field (H_{E}), a sensor (S) for measuring a magnetic field, comprising in particular at least one electrical resistor (R, R₁, R₂, R₃, R₄),
wherein
the sensor (S) is oriented vertically with respect to a direction of the generated magnetic field (H_{E}) and the sensor (S) measures local changes in the magnetic field (H_{E}) caused by the magnetically marked object (Z) in parallel with a direction of movement of the object (Z) when the object (Z) moves within the range of the sensor (S),
means (A) for providing a signal (VB, 1) of the sensor (S).

9. Device according to claim 8, wherein
the sensor (S) is embodied as a Wheatstone measuring bridge (R, R₁, R₂, R₃, R₄).

10. Use of a sensor (S) for measuring a magnetic field when magnetically marked objects (Z) move in a magnetic field (H_{E}) for detecting, in particular continuously detecting, the magnetically marked objects (Z) within the range of the sensor (S) on the basis of a threshold value (10) following conditioning (F₂, F₃) of a signal (1) generated by the sensor (S) by means of at least one convolution of the generated signal (1) using an nth derivative of a Gaussian function with n≥0, where n is a natural number including the number zero.

## Revendications

1. Procédé de détection, notamment en continu, d'objets ( Z ) caractérisés magnétiquement, notamment d'objets biologiques, de préférence de cellules, comprenant les stades de déplacement ( S₁ ) d'au moins un objet ( Z ) caractérisé magnétiquement dans un champ ( H_{E} ) magnétique,
de mesure ( S₂ ) d'une variation locale du champ ( H_{E} ) magnétique due à l'objet ( Z ) caractérisé magnétiquement,
de production ( F₁ ) d'un signal ( 1 ), notamment numérisé, à l'aide de la variation locale qui a été mesurée du champ ( H_{E} ) magnétique,
de traitement ( F₂, F₃) du signal ( 1 ) produit au moyen d'au moins d'une convolution du signal ( 1 ) produit par une fonction mathématique,
d'exploitation ( F₄ ) du signal ( 1" ) traité, l'exploitation du signal ( 1" ) comprenant la détermination ( F₄ ) d'extremums ( M₁, M₂ ), notamment de maximums, du signal ( 1" ) et la comparaison des extremums ( M₁, M₂ ) déterminés à une valeur ( 10 ) de seuil, qui est notamment donnée à l'avance et dont le dépassement vaut détection de l'objet ( Z ),
**caractérisé**
**en ce que** le traitement du signal ( 1 ) produit comprend un lissage ( F₂ ) par convolution du signal ( 1 ) produit par une fonction de Gauss.

2. Procédé suivant la revendication 1, dans lequel
on oriente le champ ( H_{E} ) magnétique sensiblement perpendiculairement à une direction du déplacement de l'objet ( Z ), des variations du champ ( H_{E} ) magnétique qui sont provoquées par des variations d'une densité de flux magnétique en raison de l'objet ( Z ) caractérisé magnétiquement étant notamment mesurées parallèlement à la direction de déplacement de l'objet ( Z ).

3. Procédé suivant au moins la revendication 1, dans lequel on mesure la variation du champ ( H_{E} ) magnétique au moyen d'un pont de Wheatstone ( R₁, R₂, R₃, R₄ ).

4. Procédé suivant au moins la revendication 1, dans lequel le traitement ( F₂, F₃ ) et/ou l'exploitation ( F₄ ) du premier signal ( 1 ) produit, comprend une convolution ( F₃ ) du signal ( 1 ) par au moins une dérivée de la fonction de Gauss d'ordre supérieur ou égal à un.

5. Procédé suivant au moins la revendication 1, dans lequel on effectue le traitement ( F₂, F₃ ) du signal ( 1 ) produit au moyen d'une vitesse ( v_{z} ) de l'objet ( Z ) et/ou de dimensions d'un capteur ( b, d ).

6. Procédé suivant au moins la revendication 1, dans lequel on adapte dynamiquement la valeur ( 10 ) du seuil, notamment par des méthodes statistiques.

7. Dispositif de détection, notamment en continu, d'objets ( Z ) caractérisés magnétiquement, notamment d'objets magnétiques, de préférence de cellules, et propre notamment à l'exécution d'un procédé suivant au moins l'une des revendications 1 à 6, comprenant
un dispositif ( 20 ) formant capteur pour déterminer, notamment en continu, des objets caractérisés magnétiquement, des moyens ( B₁ ) d'amenée d'au moins un objet ( Z ) au dispositif ( 20 ) formant capteur,
des moyens ( B₂ ) d'éloignement du au moins un objet ( Z ) du dispositif ( 20 ) formant capteur,
des moyens ( F₁, F₂, F₃) de production et de traitement d'un signal ( 1, V_{B} ) produit par le dispositif ( 20 ) formant capteur constitués pour la convolution au moins une fois du signal ( 1, V_{B} ) produit par une fonction de Gauss,
une unité ( C ) d'exploitation, qui exploite le signal ( 1" ) traité de manière à ce que l'exploitation du signal ( 1" ) comprenne la détermination ( F₄ ) d'extremums du signal ( M₁, M₂ ) du signal ( 1" ) et la comparaison des extremums ( M₁, M₂ ) déterminés à une valeur ( 10 ) de seuil, qui est notamment donnée à l'avance et dont le dépassement vaut détection de l'objet ( Z ).

8. Dispositif suivant la revendication 7,
dans lequel le dispositif ( 20 ) formant capteur comporte :
des moyens de détection d'un champ ( H_{E} ) magnétique, un capteur ( S ) de mesure d'un champ magnétique comprenant notamment au moins une résistance ( R₁, R₂, R₃, R₄ ) électrique, dans lequel
le capteur ( S ) est orienté perpendiculairement à une direction du champ ( H_{E} ) magnétique produit et le capteur ( S ) mesure parallèlement à une direction de déplacement de l'objet ( Z ), des variations locales du champ ( H_{E} ) magnétique dues à l'objet ( Z ) caractérisé magnétiquement lors d'un déplacement de l'objet ( Z ) dans la région du capteur ( S ),
des moyens ( A ) de traitement d'un signal ( VB, 1 ) du capteur ( S ).

9. Dispositif suivant la revendication 8,
dans lequel le capteur ( S ) est constitué sous la forme d'un pont ( R₁, R₂, R₃, R₄ ) de mesure de Wheatstone.

10. Utilisation d'extremums ( M₁, M₂ ) d'un signal ( 1, 1', 1", 1'" ) d'un capteur ( S ) pour la mesure d'un champ magnétique lors du déplacement d'objets ( Z ) caractérisés magnétiquement dans un champ ( H_{E} ) magnétique pour la détection, notamment en continu, des objets ( Z ) caractérisés magnétiquement dans la région du capteur ( S ) au moyen d'une valeur ( 10 ) de seuil après un traitement ( F₂, F₃) d'un signal ( 1 ) produit par le capteur ( S ), le traitement s'effectuant au moyen d'au moins une convolution du signal ( 1 ) produit par une dérivée énième d'une fonction de Gauss avec n≥ à 0, n étant un nombre en naturel, y compris le nombre zéro.
